# EUROPÄISCHE PATENTSCHRIFT

(11) **EP 2 663 585 B1**
(45) Veröffentlichungstag und Bekanntmachung des Hinweises auf die Patenterteilung: **19.04.2017**
(21) Anmeldenummer: 12706440.0
(22) Anmeldetag: 13.01.2012
(51) Int. Cl.: C08G 18/08, C08G 18/10, C08G 18/12, C08G 18/40, C08L 75/02, C08J 11/00, C08J 11/10, C08J 11/28

(54) **OLIGOHARNSTOFF-VERBINDUNGEN UND VERFAHREN ZU IHRER HERSTELLUNG UND IHRE VERWENDUNG**
OLIGOUREA COMPOUNDS AND METHOD FOR PRODUCING SAME AND USE THEREOF
COMPOSÉS D'OLIGO-URÉE, LEUR PROCÉDÉ DE PRÉPARATION ET LEUR UTILISATION

(30) Priorität: 13.01.2011 DE 102011008535
(43) Veröffentlichungstag der Anmeldung: 20.11.2013
(73) Patentinhaber: Klockemann, Werner, 21244 Buchholz (DE)
(72) Erfinder: Klockemann, Werner, 21244 Buchholz (DE)
(74) Vertreter: Müller Schupfner & Partner Patent- und Rechtsanwaltspartnerschaft mbB
(86) Internationale Anmeldenummer: PCT/DE2012/000025
(87) Internationale Veröffentlichungsnummer: WO 2012/095105

(56) Entgegenhaltungen:
- EP-A2- 0 187 333
- EP-A2- 2 103 637
- DE-A1-102009 000 604

## Beschreibung

Gegenstand der Erfindung sind Oligoharnstoff-Verbindungen, ein Verfahren zu ihrer Herstellung sowie ihre Verwendung.

Die Herstellung von Oligoharnstoff-Dispersionen durch Depolymerisation von Polyurethan-Kunststoffen, insbesondere von Polyurethan-Weichschaumstoffen, ist bekannt und wird z.B. in der WO 2009/098226 A1, der EP 2103637 A2 oder der DE 102009000604 A1 beschrieben. Nach der WO 2009/098226 A1 werden Oligoharnstoff-Teilchen mit Teilchengrößenmaxima zwischen 100 und 1000 nm bei einer Halbwertsbreite der Teilchengrößenverteilung von 100 bis 10000 nm erhalten. Bei den bisher beschriebenen Depolimerisations-Methoden werden Oligoharnstoff-Teilchen freigesetzt, die während der Schäumreaktion durch die Reaktion von Wasser mit den Isocaynaten entstehen. Deshalb findet man in der Struktur dieser Oligoharnstoff-Teilchen ausschließlich die Struktur des normalen Harnstoffs (-NH-CO-NH-) zwischen den Isocyanatresten. Der Schaumbildungsmechanismus von Poyurethan-Weichschaum ist gut aufgeklärt und führt zur Bildung von sogenannten "ureaballs" bzw "copolymer particles", die eine Größe von 200-500 nm (Größenbestimmung mittels Transmissions-Elektronenmikroskop [TEM]) besitzen (Herrington R; and Hock K; Flexible Polyurethane Foams, 2nd Ed., Midland, Michigan, The Dow Chem Co: (1998). Bei den bisher beschriebenen Depolymerisationsmethoden werden die in der Schaumstruktur gebildeten Oligoharnstoff-Teilchen (200-500nm) freigesetzt und können in den Dispersionen nachgewiesen werden. Die Herstellung von Oligoharnstoffstrukuren mit Aminharnstoffen (z.B. -NH-R₁-CH₂-R₂-NH-) zwischen den Isocyanatresten, die Herstellung kleinerer Teilchen (z.B. 1-50 nm) und/oder die Herstellung von Produkten mit engere Partikelgrößenverteilungen sind nach diesen Verfahren nicht möglich. Polyharnstoff -Dispersionen als solche sind z.B. aus der EP 0187333 A2 bekannt.

Aufgabe der Erfindung ist es, Harnstoff-Verbindungen, ggf. versehen mit reaktiven Gruppen, insbesondere dispergiert in einem Dispergiermittel sowie ein Verfahren zu ihrer Herstellung zur Verfügung zu stellen. Es ist insbesondere Aufgabe der vorliegenden Erfindung möglichst molekulareinheitliche Partikel, d.h. mit engen Teilchengrößenverteilungen, im Nanometerbereich von unter 40 nm in Dispersion und ohne Wasser zielgerichtet herzustellen.

Die molekulareinheitlichen Partikel sollen mit funktionellen, d. h. reaktiven, Gruppen ausstattbar sein, um so durch Umsetzung mit einer weiteren Reaktionskomponente zu Endprodukten verarbeitet werden zu können.

Erfindungsgemäß wird die Aufgabe gelöst durch den Gegenstand der unabhängigen Ansprüche. Weitere Ausgestaltungen, insbesondere bevorzugte Ausgestaltungen, sind Gegenstand der Unteransprüche oder nachfolgend beschrieben.

Gegenstand der Erfindung ist ein Verfahren zur Herstellung von oligomeren Harnstoff-Verbindungen durch Umsetzung von Ausgangsverbindungen mit jeweils zumindest zwei reaktiven Gruppen, ausgewählt aus Hydroxy (-OH) und/oder Thiol- (-SH-) Gruppen mit Di- oder Polyisocyanten, bei einer ersten Reaktionstemperatur, um Polyurethan- und/oder Polythiourethan-Verbindungen aufzubauen und Depolymerisation der entstandenen Polyurethan- und/oder Polythiourethan-Verbindungen in Anwesenheit eines primären oder sekundären Di- oder Polyamins bei einer zweiten Reaktionstemperatur, wobei die zweite Reaktionstemperatur, bezogen auf das Maximum der jeweiligen Temperatur, um mindestens 40°C, vorzugsweise mindestens 70°C, insbesondere mindestens 100°C höher ist, als die erste Reaktionstemperatur, um Zusammensetzungen zu erhalten, aufweisend oligomere Harnstoff-Verbindungen, die insbesondere zumindest teilweise in Teilchenform vorliegen und die Ausgangsverbindungen mit zumindest zwei reaktiven Gruppen, ausgewählt aus Hydroxy (-OH) und/oder Thiol- (-SH) -Gruppen. Die durch Depolymerisation zugänglich gemachten Ausgangsverbindungen bzw. etwaige nicht umgesetzte Ausgangsverbindungen wirken als Verdünnungs- bzw. Dispergiermittel.

Die primären oder sekundären Di- oder Polyamine und/oder zusätzliche Mono-Amine werden bevorzugt nach Bildung der Polyurethan- und/oder Polythiourethan-Verbindungen (in Bezug auf erstere ggf. teilweise) zugesetzt, aber vor oder während der Temperaturerhöhung auf die zweite Reaktionstemperatur, insbesondere vor der Temperaturerhöhung auf die zweite Reaktionstemperatur.

Dies bewirkt, dass nach Bildung der Polyurethan- und/oder Polythiourethan-Verbindungen vorzugsweise vorhandene freie Isocyanat-Gruppen mit dem Amin abreagieren, insbesondere vor der Depolymerisation. Die Depolymerisation wird dann durch die Temperaturerhöhung eingeleitet.

Die Depolymerisation durch Temperaturerhöhung bewirkt ein Spaltung der Urethanbindung, womit ohne an die Theorie gebunden zu sein, (wieder) freie Isocyanat - Bindungen entstehen, die mit dem Di- oder Polyaminen und ggf. weiter vorhandenen Mono-Aminen bei der zweiten Reaktionstemperatur reagieren.

Nach einer Ausgestaltung werden dimere, trimere und tetramere oligomere Harnstoff-Verbindungen neben monomeren Harnstoff-Verbindungen und oligomeren Harnstoff-Verbindungen mit einem Oligomerisationsgrad von 5 bis 16 erhalten. Insbesondere weisen, bezogen auf die Oligoharnstoff-Moleküle (Anzahl), ggf. ausgenommen die Monomere, mehr als 50 %, insbesondere mehr als 80 %, aller Oligoharnstoff-Moleküle Oligomerisationsgrade von 2 bis 16, insbesondere 2 bis 8 auf. Ein Oligomerisationsgrad von 2 bedeutet, dass 2 Momomereinheiten miteinander verknüpft sind, z.B. ein Diamin mit einem Isocyanat.

Die Herstellung der Polyurethan- und/oder Polythiourethan-Verbindungen erfolgt vorzugsweise in Abwesenheit von anderen Stoffen, wie diese typischerweise in Polyurethanreaktionen zugesetzt werden, wie Wasser, Katalysatoren (tertiäre Amin- und Zinnkatalysatoren), Farbstoffe, Stabilisatoren (wie Silikone) und/oder Treibmittel.

Die als Zwischenprodukt erhaltenen Polyurethan- und/oder Polythiourethan-Verbindungen werden nicht in fester Form erhalten bzw. durchlaufen keinen festen Zustand sondern liegen während und unter den Bedingungen der Umsetzung stets als Flüssigkeit, in einer Flüssigkeit dispergiert oder in gelöster Form vor und/oder die beide Umsetzungen, Kettenaufbau und Depolymerisation, werden in einem einzigen Reaktor als Eintopfreaktion durchgeführt.

Nach einer Ausführungsform der Erfindung wird ein Dispergiermittel zumindest während der Depolymerisation zugesetzt. Nach einer anderen Variante wird auf die Zugabe zusätzlichen Dispergiermittels oder Lösemittels verzichtet und die Depolymerisation erfolgt ausschließlich in Gegenwart der Spaltprodukte als Dispergiermittel oder Lösemittel.

Die Umsetzungen können in üblichen Rührreaktoren, in Dispergatoren, Schnellmischern, Düsenstrahldispergatoren, Reaktionsextrudern, Extrudern oder Mischerknetern durchgeführt werden.

Bei der Herstellung der Oligoharnstoff-Dispersionen wird in einem ersten Schritt ein Diisocyanat, vorzugsweise im molaren Überschuss, mit einer für den späteren Verwendungszweck als geeignet ausgewählten Ausgangsverbindung mit zumindest zwei Gruppen, ausgewählt aus -OH und/oder -SH, in Kontakt gebracht und gegen Ende der Umsetzung des Diisocyanats mit der Ausgangsverbindung zu dem Vorpolymerisat ein Amin oder ein Amin-Gemisch aufweisend primäre und/oder sekundäre Amin-Gruppen zugegeben und zur Reaktion gebracht wird und durch die durch Temperaturerhöhung bewirkte Spaltung der Polyurethan-/ Polythiourethan-Bindung Harnstoff-Bindungen bzw. Harnstoff-Verbindungen entstehen, deren Größe durch das molare Verhältnis von Isocyanat zur Ausgangsverbindung und die Art des Amins bestimmt werden. Die Harnstoff-Verbindungen sind in der freigesetzten Polyhydroxy-/Polythio-Verbindung und dem fakultativen Dispersionsmittel als nanoskalige Teilchen dispergiert.

Die Umsetzung zur Polyurethan- bzw. Polythiourethan-Verbindung erfolgt z.B. für 10 Minuten bis 8 Stunden und unabhängig hiervon bei Temperaturen zwischen 20 und 120°C. Das so hergestellte flüssige Vorpolymerisat wird unmittelbar anschließend oder nach einer beliebigen Zeit mit einer Amin-Verbindung, ggf. in Gegenwart eines Dispergiermittels und unter Bedingungen in Kontakt gebracht, unter denen die Isocyanat-Gruppe sich mit den Amin-Gruppen der Amin-Verbindung umsetzt und die Urethan-/Thiourethan-Gruppen (nach Spaltung), zumindest teilweise, ggf. auch vollständig, mit den Amingruppen zu Harnstoffgruppen, unter Freisetzung der bei der Synthese des Vorpolymerisats verwendeten Ausgangsverbindung bei Temperaturen von 120 bis 250°C, insbesondere 120 bis 220°C, insbesondere 120 bis 180°C, gespalten werden. Die Harnstoff-Verbindungen können sich in dem Dispersionsmittel als nanoskalige Teilchen dispergieren.

Die erfindungsgemäß ggf. zusätzlich einzusetzenden Dispergiermittel können bevorzugt sein Diole einschließlich Polyetheralkoholen. Die Diole können einfache Diole wie Ethylenglykol, Diethylenglykol, Triethylenglykol, Tetraethylenglykol, höhere Polyethylenglykole, Propylenglykol, Dipropylenglykol, Tripropylenglykol, Tetrapropylenglykol, höhere Propylenglykole, Butan-1,4-diol, α,ω-Bishydroxy-butylenglykole, Copolymere des Ethylenoxids und Propylenoxids sein, wobei die langkettigen Diole Molmassen bis zu 6000 aufweisen können.

Die Polyetheralkohole können di- oder polyfunktionell sein, im allgemeinen werden die typischen Polyetheralkohole der Polyurethanchemie verwendet, z.B. Polyethertriole mit Glycerin oder Trimethylolpropan als Starter und Propylenoxid und ggf. Ethylenoxid statistisch verteilt oder als innerer oder Endblock, wobei die Molmasse zwischen 400 und 6000 liegt.

Das zusätzlich einzusetzende Dispergiermittel wird, wenn es für die Isocyanat-Gruppe reaktive Gruppen aufweist, erst zur Depolymerisationsreaktion zugesetzt. Grundsätzlich sind als Dispergiermittel auch andere Verbindungen geeignet, wenn sie die Oligoharnstoff-Moleküe nicht lösen, wie z.B. inerte Verbindungen ohne reaktive Gruppen.

Erfindungsgemäß einsetzbare Amine bzw. Amin-Gemische sind
a) Verbindungen, aufweisend in der Summe zumindest zwei primäre, zwei sekundäre oder zumindest eine primäre und zumindest eine sekundäre Amin-Gruppe
und ggf. zusätzlich
b) primäre oder sekundäre Monoamine, die ggf. weitere funktionelle Gruppen tragen können, wie -OH, -SH, die jeweils unter den Bedingungen der Spaltung nicht reagieren
c) Ammoniak und ggf. Wasser.

Geeignete Amine der Gruppe a) sind z.B. Harnstoff, Hexan-1,6-diamin, Di-iso-propylamin, Ethylendiamin, N,N'-Dimethyl-ethylendiamin, 1,3-Propylendiamin, Isophorondiamin, 4,4'-Diaminodicyclohexylmethan, Diethylentriamin, Triethylentetramin, N,N-Bis-(2-aminopropyl)methylamin, N,N-Bis-(3-aminopropyl)methylamin, Dipropylentriamin, Tripropylentetramin, 1,4-Phenyl-endiamin, Guanidin, Poly-Guadine, 1,3-Phenylendiamin, 4,4'-Diaminodiphenylmethan, Triaminnonan usw. Weiterhin geeignet sind AminoGruppen enthaltende Verbindungen, einschließlich Polymeren wie α,ω-Diaminopolyether, Mannichbasen oder Oligoethylenimine. Insbesondere α,ω-Diaminopolyether auf der Basis von Bis-hydroxypropylenglykolen der Molmasse 200 bis 2000 können verwendet werden.

Im Sinne der vorliegenden Erfindung wird Harnstoff als Verbindung mit zwei primären Amin-Gruppe aufgefasst und der Grupp a) zugeordnet.

Geeignete Amine der Gruppe b) sind Di-n-butylamin oder Di-iso-butylamin. Zur Herstellung von hydroxylfunktionellen nanoskaligen Harnstoffen in funktionellen Dispersionsmitteln zu den erfindungsgemäßen funktionellen Harnstoff-Nanodispersionen können Alkanolamine als Kettenunterbrecher eingesetzt werden, bei denen die Aminogruppe mit den Isocyanaten zu Harnstoffgruppen reagiert und die weniger reaktive Hydroxylgruppe (zumindest in Gegenwart von Aminen) frei verfügbar bleibt. Auf diesem Wege werden hydroxylfunktionelle Harnstoff-Nanodispersionen hergestellt. Geeignete Alkanolamine sind z.B. Ethanolamin, Diethanolamin, N-Methylethanolamin, 2- oder 3-Propanolamin, Dipropamolamin, N-Methyl-propanolamin etc.

Andere geeignete Verbindungen, die mittels einer oder mehrerer Amingruppen eingeführt werden können, sind Verbindungen, enthaltend zumindest eine primäre oder sekundäre Amin-Gruppe und weiterhin aufweisend eine Phosphatgruppe (z.B. zur Verwendung als Flammschutzmittel) oder Verbindungen, enthaltend zumindest eine primäre oder sekundäre Amin-Gruppe und weiterhin aufweisend eine Schwefelgruppe (z.B. zur Verwendung als Fungizid oder Biozid). Ein Beispiel für eine Verbindung mit zwei primären Amin-Gruppen ist Guanidin (die =NH Gruppe ist im Rahmen der Depolymerisationsreaktion nicht oder wenig reaktiv), ein Bespiel mit zwei sekundären Amin-Gruppen ist 2-Methylthio-4-*tert-*butylamino- 6-cyclopropylamino-s-triazin (CAS: 28159-98-0, Handelsnamen: Irgarol 1051 oder Cybutryn). Hierbei wird die als Biozid wirksame Gruppe in die Kette und nicht nur an das Kettenende eingebaut.

Es können somit Oligomere mit dauerhaft bioziden Eigenschaften hergestellt werden, indem eine oder mehrere biozid wirksame Verbindungen mit jeweils mindestens zwei chemisch reaktiven Gruppen der Oligoharnstoffe, z.B. im molaren Verhältnis 1 : 1 bis 1 : 4, sowie ggf. weiteren Kettenverlängerern, Spacern und/oder Vernetzern mit mindestens drei reaktiven Gruppen umgesetzt werden. Durch die Umsetzung werden Polymere mit dauerhaft bioziden Eigenschaften hergestellt, die Struktureinheiten aufweisen aus einer oder mehreren biozid wirksamen Verbindungen mit mindestens zwei chemisch reaktiven Gruppen und einer zweiten, langkettigen Einheit mit mindestens zwei mit den reaktiven Gruppen reaktionsfähigen und umgesetzten Gruppen sowie ggf. weiteren Kettenverlängerern, Spacern und/oder Vernetzern mit mindestens drei reaktiven Gruppen.

Es wurde überraschend gefunden, dass bestimmte Biozidverbindungen ihre biozide Wirkung auch dann behalten, wenn sie in eine Oligomerhauptkette eingebunden werden. So kann beispielhaft das als Irgarol® 1051 bezeichnete 2-Methylthio-4-*tert*-butylamino- 6-cyclopropyl-amino-s-triazin über die beiden NH-Gruppen mit Di- und/oder Polyisocyanaten bzw. mit Vorpolymerisaten (Prepolymeren) auf deren Basis reagieren und in Abhängigkeit von der Struktur der Isocyanatkomponente und der Polyolkomponente elastische, halbharte oder harte Poly(urethan-harnstoffe) ergeben, die durch den Einbau der Verbindung durchgängig biozid gegenüber Algen, Bakterien und Mikropilzen (Fungi) sind. 2-Methylthio-4-*tert*-butylamino-6-cyclopropylamino-*s*-triazin ist nur ein Beispiel für ein biozidwirksames Molekül, das außer über eine biozide Wirkung auch ein hochwirksamer Baustein im Polyadditionsverfahren mit Di- und/oder Polyisocyanaten ist.

Weitere als Biozid wirksame Verbindungen, die in dem erfindungsgemäßen Verfahren zur Herstellung der erfindungsgemäßen dauerhaft biozid wirksamen Polymeren eingesetzt werden können, sind Carbendazim [(N-(Benzimidazol-2-yl)carbamidsäuremethyl-ester], N-(3-Aminopropyl)-N-dodecylpropan-1,6-diamin, N-Dodecyl-propylendiamin, 5-Chlor-2-methyl-isothiazolinon, 2-Octyl-3(2H)-isothiazolinon, Methyloxazolidin, Dichloroctylisothiazolinon (Vinyzene DCOIT), Octylisothiazolinon, N,N'-Diethylpiperazin, N,N'-Dibenzylpiperazin, N,N'-Dicyclohexyl-piperazin, Piperidin, sowie andere substituierte Oxazolidine, Benzimidazole, Piperazine, Piperidine oder Isothiazoline. Die eingebaute Menge an biozid wirksamen Verbindungen beträgt erfindungsgemäß zwischen 0,01 bis 10 Gew.% der so erhaltenen Zusammensetzung.

Geeignete Di- oder Polyisocyanate sind alle an sich bekannten aliphatischen, cycloaliphatischen, araliphatischen oder aromatischen Isocyanate, wobei die aromatischen Di- oder Polyisocyanate bevorzugt werden. Beispiele sind 4,4'-Diphenylmethandiisocyanat, 1,4-Phenylen-diisocyanat, 1,4-Xylylendiisocyanat, Toluylen-1,4-diisocyanat, Toluylen-1,6-diisocyanat, 2,4'-Diphenylmethandiisocyanat, 2,2'-Diphenylmethandiisocyanat, Hexan-1,6-diisocyanat, Iso-phorondiisocyanat, Tetramethylxylylendiisocyanat, 4,4'-Dicyclohexyl-methandiisocyanat, Triisocyanatononan, Cyclohexan-1,4-diisocyanat usw.

Die Di- oder Polyhydroxy-Verbindung ist insbesondere ein Diol, kann z.B. aber auch ein Triol oder Tetrol sein, bzw. deren Gemische. Als Polyhydroxy-Verbindung sind Polyetheralkohole bevorzugt.

Neben oder statt den Aminen, die zur Abreaktion der überschüssigen Isocyant-Gruppen nach der Kettenaufbaureaktion eingesetzt werden, können auch Wasser und/oder Ammoniak verwendet werden.

Die erfindungsgemäßen Oligoharnstoff-Dispersionen enthalten ein Dispergier-oder Verdünnungsmittel, und weisen
- Oligoharnstoff-Moleküle mit 2 bis 16, vorzugsweise 2 bis 8 Monomereinheiten, auf,
   insbesondere im Mittel (ggf. ausgenommen Monomere) mit 2 bis 16, vorzugsweise im Mittel 2 bis 8, Monomereinheiten,
   wobei insbesondere mehr als 50 %, insbesondere mehr als 80 %, aller Harnstoff-/Oligoharnstoff-Moleküle (ggf. ausgenommen die Monomere) Oligomerisationsgrade von 2 bis 16, insbesondere 2 bis 8, insbesondere bevorzugt 2 bis 6 aufweisen.

Die Oligoharnstoff-Moleküle in Dispersion lassen sich insbesondere durch eines oder mehrere der folgenden Merkmale charakterisieren:
a) die Oligoharnstoff-Moleküle bilden in dem Dispergiermittel Teilchengrößen von im Mittel 4 bis 40 nm, vorzugsweise 8 bis 20 nm aus.
b) die Oligoharnstoff-Moleküle bilden in dem Dispergiermittel Teilchengrößen von kleiner 40 nm für 90% aller Teilchen, insbesondere von kleiner 30 nm für 90% aller Teilchen und besonderes bevorzugt von kleiner 20 nm für 90% aller Teilchen (jeweils wie durch Laser-Lichtstreuung (Zetasizer S, Malvern gemessen) und/oder.
c) die Oligoharnstoff-Moleküle bilden in dem Dispergiermittel insbesondere Teilchengrößenverteilungen wonach 80% aller Teilchen, insbesondere 90%, aller Teilchen, eine Teilchengröße von minus 10 bis plus 10 nm um den Mittelwert aufweisen.

Insbesondere liegen die Oligoharnstoff-Verbindungen als Partikel in Dispersion vor. Nach einer anderen Alternative liegen diese ganz oder teilweise in Lösung vor.

Die Endgruppen können sein, mindestens eine freie Amino- und/oder Hydroxylgruppe, Carboxyl-, oder SH-Gruppe (z.B. zwei Aminogruppen, zwei Hydroxylgruppen oder eine Amino- und eine Hydroxylgruppe) als Endgruppe.

Die nm-Angaben beziehen sich jeweils auf den Partikeldurchmesser gemäß hydrodynamischem Volumen und wie durch Laser-Lichtstreuung (Zetasizer S, Malvern) in der jeweiligen Dispersion bestimmt.

Das Dispergiermittel kann ebenfalls funktionelle Gruppen, vorzugsweise pro Molekül zwei freie/funktionelle Gruppen, aufweisen, ausgewählt aus der Gruppe-OH, -NH₂, =NH, vorzugsweise ganz oder teilweise -OH.

Die funktionellen Gruppen der Oligoharnstoff-Teilchen und der/des Dispersionsmittel(s) sind vorzugsweise so ausgewählt, dass sie in der funktionellen Harnstoff-Nanodispersion, zumindest nach Abschluss der Reaktion, nicht miteinander reagieren.

Insbesondere enthalten oder bestehen die erfindungsgemäßen Oligoharnstoff-Dispersionen aus:
- 1 bis 90 Gew.-%, oder 5 bis 50 Gew.-% Mono- und/oder Oligoharnstoff-Molekülen,
- 99 bis 10 Gew.-%, oder 95 bis 50 Gew.-%, eines Dispergiermittels, welches mindestens eine -OH und/oder -SH Gruppe aufweist.

Die erfindungsgemäß verwendbaren Mono- und Oligoharnstoff-Moleküle bestehen z.B. aus den Grundkörpern geeigneter Di- und/oder Polyisocyanate, die mit Mono-, Di- und/oder Polyaminen umgesetzt wurden und demzufolge als di-und/oder trisubstituierte Harnstoffe vorliegen, wobei an den Harnstoffen vorzugsweise eine Endgruppe eine primäre oder sekundäre Aminogruppe ist.

Diese Harnstofftmoleküle können allgemein in Bezug auf die Umsetzung mit einem Diisocyanat und einem Diamin dargestellt werden als:

H₂NR-X(-R')-NH-CO-NH-R"-NH-CO-NH-X(-R')-RNH₂ (Monomer)

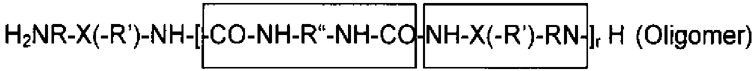

mit
R': Wasserstoff, ein aliphatischer oder aromatischer Rest,
X: eine -CH₂-, -CH=, -C₂H₄-, -C₃H₈- oder höhere aliphatische Gruppe, eine - CₙHₙ₊₂NH-Gruppe, eine -CₙHₙ₊₂N(CₙHₙ₊2)ₘNH- Gruppe, und
R": ein aliphatischer C₄-bis C₁₆-Rest, ein aromatischer Rest, ein Biphenylrest, ein Diphenylmethanrest.
n: 1 bis 18 und m = 1 oder 2 und
r: 2 bis 20.

Durch die nachfolgenden Versuchsbeispiele wird die Erfindung erläutert, ohne auf diese beschränkt zu sein.

### Beispiele

### Analytik

Die Teilchengröße wurde mittels Laser Lichtstreuung (Nanophox® der Sympatec GmbH (PCCS), Zetasizer S, Malvern GmbH) in Dispersion, in dem sich jeweils aus der Umsetzung ergebenden Medium bestimmt. Das Maximum der Verteilungskurve ist in nm angegeben (Durchmesser gemäß hydrodynamischem Volumen).

Die Messung der Partikelgrößen erfolgte nach 2 unterschiedlichen Methoden an 2 Gerätesystemen: Zetasizer S (Firma: Malvern) und Nanophox (Firma: Sympatec). Beide Geräte nutzen das Prinzip der dynamischen Lichtstreuung zur Partikelgrößenbestimmung.

Im Gerätesystemen, die mittels dynamischer Lichtstreuung messen, erfolgt eine Messung durch Durchstrahlung der Probe mit einem Laser. Durch Lichtstreuung entsteht auf dem Detektorfenster ein Interferenzmuster aus hellen und dunklen Punkten. Kleine Partikel weisen gegenüber großen Partikeln eine höhere Beweglichkeit auf und verursachen über einen definierten Zeitraum einen schneller fluktuierenden Hell-Dunkel-Zustand auf dem Bildschirm. Anhand der Geschwindigkeit der Fluktuation der Helligkeitspunkte kann auf die Teilchengröße der Partikel geschlossen werden (Stokes-Einstein-Beziehung).

Im Nanophox-Gerätesystem wird die Photon-Crosscorrelation -Spectroscopy (PCCS)-Technik zur Messung/Auswertung eingesetzt. Die PCCS ist eine Technik, die es erlaubt gleichzeitig Messungen der Partikelgröße und der Stabilität im Nano- bis Mikrometerbereich in Suspensionen und Emulsionen zu machen. Das Schlüsselprinzip der PCCS ist eine 3D-Kreuzkorrelationstechnik. Durch eine spezielle Streugeometrie ist die Kreuzkorrelation des gestreuten Lichtes in der Lage, die Fraktion des einfach gestreuten Lichtes von der des mehrfach gestreuten Lichtes präzise zu trennen. Das Gerät arbeitet mit zwei separaten Laserstrahlen und zwei Detektoren.

Die Teilchengrößenverteilung wie mit dem Zetasizer S bestimmt ist graphisch dargestellt (jeweils Intensität in % gegen Teilchendurchmesser in nm). Es zeigen:
- Fig.1: Die Teilchengrößenverteilung (Durchmesser) der Teilchen in der Zusammensetzung des Beispiels 6 gemessen in der dort erhaltenen Dispersion
- Fig.2: Die Teilchengrößenverteilung (Durchmesser) der Teilchen in der Zusammensetzung des Beispiels 7 gemessen in der dort erhaltenen Dispersion

Beide Messungen erfolgen jeweils in dem Dispergiermittel bei 40° und wie es jeweils als Ergebnis der Umsetzung vorlag. Die Größe von MDA wird mit 1,8 nm angenommen. Ein Produkt aus 1 Molekül MDI und 2 Molekülen DETA wurde auf 2,5 nm kalkuliert. Eine gemessene Partikelgröße von 10 nm könnte somit theoretisch ein Tetramer dieser Struktur sein, 20nm-Partikel könnten theoretisch ein Oktamer dieser Struktur sein usw. Die Viskosität wurde als kinematische Viskosität durch Rotation jeweils bei 25°C und nach DIN 53019 (Gerät: Rheostress 300) bestimmt. Der Isocyanatgehalt wurde nach DIN 53185 bestimmt, die OH-Zahl nach DIN 53240 und die Aminzahl nach DIN 16945.

Eingesetzte Edukte
- DMD:: 4,4'-Diphenylmethandiisocyanat in Schuppenform
- Lupranol® 1100:: Polypropylenglykol (Diol) mit einem mittleren Molekulargewicht von 1100 g/mol, OH-Zahl 104, Elastogran AG
- Lupranol® 1000:: Polypropylenglykol (Diol) mit einem mittleren Molekulargewicht 2000 g/mol, OH-Zahl 55, Elastogran AG
- DPG:: Dipropylenglykol
- DEG:: Diethylenglykol
- DETA:: Diethylentriamin
- DBA:: Di-n-butylamin
- DPTA:: Dipropylentriamin
- PTMO 1000:: Poly(tetramethylenoxid)Diol mit mittlerem Molekulargewicht von 1000 g/mol
- BD14:: Butan-1,4-diol
- TCD:: Tricyclo-Diamin-decan-diamin
- PC-Amin® DA145:: Bis-N,N-(2-aminopropyl)methylamin, Performance Chemicals GmbH
- Lupranol® 3403:: Polypropylenglykol auf Zuckerbasis mit einem mittleren Molekulargewicht von 550 g/mol, OH-Zahl: 445, Elastogran AG
- Lupranol® 1200:: Polypropylenglykol mit einem mittleren Molekulargewicht von 450 g/mol, Diol, OH-Zahl: 248, Elastogran AG
- PolyTHF 650 S:: Polytetrahydrofuran mit 2 Hydroxygruppen und einem mittleren Molekulargewicht von 650 g/mol, BASF AG
- Vorastar HB 6549:: Dow Chemicals Präpolymer mit Rest-NCO von 15,8%, Polymerkette Polycarprolactam
- NAEP:: N-Aminoethylpiperazin
- Lupranol® 2032:: Polypropylenglykol (Triol) mit einem mittleren Molekulargewicht von 3100 g/mol, OH-Zahl 55, Elastogran AG

### Ausführungsbeispiele

### Beispiel 1

Synthese des Vorprodukts (1. Stufe): In einen 10 l doppelwandigen Edelstahlreaktor mit Rührer, Beheizung mit Thermalöl, Dosierungen für Flüssigkeiten und Feststoffe, Stickstoffeinleitung und Wärmetauscher wurden 2,5 kg DMD gegeben, auf 45°C erwärmt und aufgeschmolzen. Zu dem flüssigen Isocyanat wurden langsam 5,5 kg Lupranol® 1100 unter Rühren zugegeben, so dass die Temperatur 55°C nicht überstieg. Das Gemisch wurde anschließend bei 46°C noch ca. 1 h gerührt. Es entstand ein homogenes, zähfließendes, gelbliches Produkt mit dem Isocyanatgehalt 5,32 %.

Umsetzung des Vorproduktes (2.Stufe): In einen 20 l Edelstahlreaktor mit Rührer, Beheizung mit Thermalöl, Dosierungen für Flüssigkeiten und Feststoffe, Stickstoffeinleitung und Wärmetauscher wurden 3,2 kg DPG, 0,8 kg DETA und 0,5 kg DBA eingewogen und dieses Gemisch unter Rühren auf 160°C erwärmt. Innerhalb von 40 Minuten wurden 5,5 kg des oben hergestellten Vorprodukts zugegeben.

Nach beendeter Zugabe wurde das Gemisch bei 180°C weitere 45 min gerührt. Das Reaktionsgemisch wurde mittels eines Bodenventils abgelassen. Es war homogen, leicht fließend, braun-orange und klar. Die Nanodispersion enthielt 31,4 Gew,% Oligoharnstoffe mit Aminendgruppen. Die Bestimmung der Teilchengröße von aminfunktionellen Oligoharnstoffen der Nanodispersion mittels Nanophox® (Sympatec GmbH, PCCS) und Zetasizer S (Malvern) ergab das Maximum der Teilchengrößenverteilungskurve (Durchmesser gemäß hydrodynam. Vol.) bei 12 nm und eine Verteilung von 10 bis 14 nm. Die Nanodispersion wies eine Hydroxylzahl von 410 mg KOH/g, eine Aminzahl von 92 mg KOH/g und eine Viskosität (Rotation) von 720 mPas (25°C) auf.

### Beispiel 2

Das im Beispiel 1 beschriebene Verfahren wurde als kontinuierliches Verfahren in einem Reaktionsextruder durchgeführt. Dabei erfolgte zunächst die Synthese des Vorproduktes unter gleichmäßiger Dosierung und Mischung des aufgeschmolzenen Diisocyanats und des Diols (molares Verhältnis 2 : 1) mit 5,0 kg 4,4'-DMD und 11,0 kg Lupranol® 1100) pro Stunde bei 45°C bis 70°C (Temperaturgradient) im vorderen Teil des Extruders. Die Umsetzung des entstandenen Vorproduktes erfolgte direkt im Anschluss im zweiten Teil des Extruders (Heizzonen 4 bis 8) bei 180°C unter Dosierung des vorgemischten Solvolysegemisches (32 Teile DPG, 8 Teile DETA und 5 Teile DBA). Das Produkt war homogen, fließfähig, braun-orange und klar. Die Nanodispersion enthielt 31,4 Gew.% Oligoharnstoffe. Die Bestimmung der Teilchengröße von aminfunktionellen Oligoharnstoffen in der Nanodispersion mittels Nanophox® (Sympatec GmbH, PCCS) und Zetasizer S (Malvern) ergab das Maximum der Teilchenverteilungskurve bei 12 nm und eine Verteilung von 10 bis 14 nm. Die Nanodispersion wies eine Hydroxylzahl von 425 mg KOH/g, eine Aminzahl von 95 mg KOH/g und eine Viskosität (Rotation) von 720 mPas (25°C) auf.

### Beispiel 3

Synthese des Vorprodukts (1. Stufe); In einen 10 l Edelstahlreaktor mit Rührer, Beheizung mit Thermalöl, Dosierungen für Flüssigkeiten und Feststoffe, Stickstoffeinleitung und Wärmetauscher wurden 2,7 kg DMD auf 45°C erwärmt und in dieses langsam unter Rühren 5 kg PTMO 1000 (ca. 1,5 Stunden) gegeben, so dass die Temperatur 75°C nicht überstieg. Das Gemisch wurde anschließend bei 60°C ca. 1h gerührt. Es entstand ein homogenes, zähfließendes, gelbliches Produkt mit einem Isocyanatgehalt von 5,5 %.

Umsetzung des Vorproduktes (2.Stufe): 5,5 kg dieses Vorproduktes wurden über eine Bodenablassleitung direkt in einen 20 l Edelstahlreaktor mit Rührer, Beheizung mit Thermalöl, Stickstoffeinleitung und Wärmetauscher mit einem auf 120°C erwärmten Gemisch von 3,2 kg DPG, 0,9 kg DPTA und 0,5 kg DBA gegeben und nach vollständiger Zugabe dieses Gemisch unter Rühren auf 180°C erwärmt. Bei 180°C wurde 30 Minuten weiter gerührt, danach wurde das Reaktionsgemisch mittels eines Bodenventils abgelassen. Es war homogen, fließend und klar. Die Nanodispersion enthielt 19,2 Ges.% aminfunktionelle Oligoharnstoffe. Die Bestimmung der Teilchengröße von aminfunktionellen Oligoharnstoffen in der Nanodispersion mittels Zetasizer (Malvern) ergab das Maximum der Teilchengrößenverteilungskurve bei 11 nm. Die Nanodispersion wies eine Hydroxylzahl von 340 mg KOH/g, eine Aminzahl von 92 mg KOH/g und eine Viskosität (Rotation) von 400 mPas (25°C) auf.

### Beispiel 4

Synthese des Vorprodukts (1. Stufe): In einen 10 l doppelwandigen Edelstahlreaktor mit Rührer, Beheizung mit Thermalöl, Dosierungen für Flüssigkeiten und Feststoffe, Stickstoffeinleitung und Wärmetauscher wurden 5 kg DMD gegeben, auf 45°C erwärmt und aufgeschmolzen. Zu dem flüssigen Isocyanat wurden langsam 4,5 kg Lupranol® 1200 unter Rühren zugegeben, so dass die Temperatur 55°C nicht überstieg. Das Gemisch wurde anschließend bei 46°C noch ca. 1h gerührt. Es entstand ein homogenes, zähfließendes, gelbliches Produkt mit dem Isocyanatgehalt 6,8 %.

Umsetzung des Vorproduktes (2.Stufe): In einen 20 l Edelstahlreaktor mit Rührer, Beheizung mit Thermalöl, Dosierungen für Flüssigkeiten und Feststoffe, Stickstoffeinleitung und Wärmetauscher wurden 2,1 kg DPG, 1,5 kg DETA und 0,4 kg DBA und 1 kg BD14 eingewogen und dieses Gemisch unter Rühren auf 160°C erwärmt. Innerhalb von 40 Minuten wurden 5 kg des oben hergestellten Vorprodukts zugegeben. Nach beendeter Zugabe wurde das Gemisch bei 180°C weitere 45 min gerührt. Das Reaktionsgemisch wurde mittels eines Bodenventils abgelassen. Es war homogen, leicht fließend, gelb und klar. Die Nanodispersion enthielt ca. 50% Oligoharnstoffe mit Aminendgruppen. Die Bestimmung der Teilchengröße von aminfunktionellen Oligoharnstoffen in der Nanodispersion mittels Zetasizer (Malvern) ergab das Maximum der Teilchengrößenverteilungskurve bei 3,5 nm und eine Verteilung von 2 bis 6 nm. Die Nanodispersion wies eine Hydroxylzahl von 568 mg KOH/g, eine Aminzahl von 187 mg KOH/g und eine Viskosität (Rotation) von 909 mPas (25°C) auf.

### Beispiel 5

Synthese des Vorprodukts (1. Stufe): In einen 10 l Edelstahlreaktor mit Rührer, Beheizung mit Thermalöl, Dosierungen für Flüssigkeiten und Feststoffe, Stickstoffeinleitung und Wärmetauscher wurden 3,0 kg DMD auf 45°C erwärmt und dabei aufgeschmolzen und dieses langsam unter Rühren mit 3,9 kg PolyTHF 650 S vermischt, so dass die Reaktionstemperatur 60°C nicht überstieg. Das Gemisch wurde anschließend bei 55°C ca. 1 h gerührt. Es entstand ein homogenes, zähfließendes, gelbliches, leicht trübes Produkt mit einem Isocyanatgehalt von 7,0 %.

Umsetzung des Vorproduktes (2.Stufe): In einen 20 l Edelstahlreaktor mit Rührer, Beheizung mit Thermalöl, Dosierungen für Flüssigkeiten und Feststoffe, Stickstoffeinleitung und Wärmetauscher wurden 2,0 kg DPG, 1,2 kg DEG, 0,9 kg PC-Amin® DA145 und 0,5 kg DBA eingewogen und dieses Gemisch unter Rühren auf 160°C erwärmt. Innerhalb von 60 Minuten wurden 5,5 kg des oben hergestellten Vorproduktes zugegeben. Nach beendeter Zugabe wurde das Gemisch bei 180°C weitere 30 min gerührt. Das Reaktionsgemisch wurde mittels eines Bodenventils abgelassen. Es war homogen, fließend und bei 35°C klar. Die Nanodispersion enthielt 24,2 Gew.% aminfunktionelle Oligoharnstoffe. Die Bestimmung der Teilchengröße von aminfunktionellen Oligoharnstoffen in der Nanodispersion mittels Zetasizer (Malvern) ergab das Maximum der Teilchengrößenverteilungskurve bei 5,8 nm. Die Nanodispersion wies eine Hydroxylzahl von 422 mg KOH/g, eine Aminzahl von 127 mg KOH/g und eine Viskosität (Rotation) von 590 mPas (25°C) auf.

### Beispiel 6

Synthese des Vorprodukts (1. Stufe): In einen 20 I doppelwandigen Edelstahlreaktor mit Rührer, Beheizung mit Thermalöl, Dosierungen für Flüssigkeiten und Feststoffe, Stickstoffeinleitung und Wärmetauscher wurden 5 kg DMD gegeben, auf 45°C erwärmt und aufgeschmolzen. Zu dem flüssigen Isocyanat wurden langsam 6,5 kg Poly-THF 650S unter Rühren zugegeben, so dass die Temperatur 55°C nicht überstieg. Das Gemisch wurde anschließend bei 46°C noch ca. 1 h gerührt. Es entstand ein homogenes, zähfließendes, gelbliches Produkt mit dem Isocyanatgehalt 7 %.

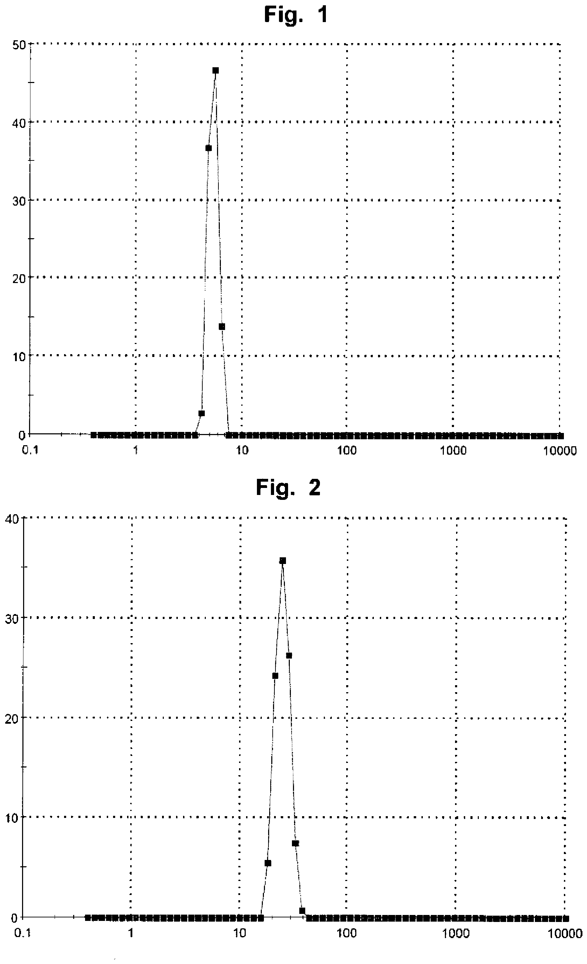

Umsetzung des Vorproduktes (2.Stufe): In einen 20 l Edelstahlreaktor mit Rührer, Beheizung mit Thermalöl, Dosierungen für Flüssigkeiten und Feststoffe, Stickstoffeinleitung und Wärmetauscher wurden 3,8 kg DPG, 0,7 kg DETA und 1,2 kg Lupranol 2032 eingewogen und dieses Gemisch unter Rühren auf 160°C erwärmt. Innerhalb von 40 Minuten wurden 4,3 kg des oben hergestellten Vorprodukts zugegeben. Nach beendeter Zugabe wurde das Gemisch bei 180°C weitere 45 min gerührt. Das Reaktionsgemisch wurde mittels eines Bodenventils abgelassen. Es war homogen, leicht fließend, gelb und klar. Die Nanodispersion enthielt 31,4 Gew.% Oligoharnstoffe mit Aminendgruppen. Die Bestimmung der Teilchengröße von aminfunktionellen Oligoharnstoffen in der Nanodispersion mittels Zetasizer (Malvern) ergab das Maximum der Teilchengrößenverteilungskurve bei 5,4 nm und eine Verteilung von 4 bis 8 nm. Die Teilchengrößenverteilungskurve ist in Fig. 1 dargestellt. Die Nanodispersion wies eine Hydroxylzahl von 416 mg KOH/g, eine Aminzahl von 74 mg KOH/g und eine Viskosität (Rotation) von 1690 mPas (25°C) auf.

### Beispiel 7

Synthese des Vorprodukts (1. Stufe): In einen 20 l doppelwandigen Edelstahlreaktor mit Rührer, Beheizung mit Thermalöl, Dosierungen für Flüssigkeiten und Feststoffe, Stickstoffeinleitung und Wärmetauscher wurden 5 kg DMD gegeben, auf 45°C erwärmt und aufgeschmolzen. Zu dem flüssigen Isocyanat wurden langsam 6,5 kg Poly-THF 650S (MG 650) unter Rühren zugegeben, so dass die Temperatur 55°C nicht überstieg. Das Gemisch wurde anschließend bei 46°C noch ca. 1 h gerührt. Es entstandt ein homogenes, zähfließendes, gelbliches Produkt mit dem Isocyanatgehalt 7 %.

Umsetzung des Vorproduktes (2.Stufe): In einen 20 l Edelstahlreaktor mit Rührer, Beheizung mit Thermalöl, Dosierungen für Flüssigkeiten und Feststoffe, Stickstoffeinleitung und Wärmetauscher wurden 3,7 kg Dipropylenglykol, 1 kg Diethylentriamin 0,4 kg Dibutylamin und 0,9 kg Polypropylenglykol MG 3100 (z.B. Lupranol 2032, Elastogran AG) eingewogen und dieses Gemisch unter Rühren auf 160°C erwärmt. Innerhalb von 40 Minuten wurden 4 kg des oben hergestellten Vorprodukts zugegeben. Nach beendeter Zugabe wurde das Gemisch bei 180°C weitere 45 min gerührt. Das Reaktionsgemisch wurde mittels eines Bodenventils abgelassen. Es war homogen, leicht fließend, gelb und klar. Die Nanodispersion enthielt ca. 27% Oligoharnstoffe mit Aminendgruppen. Die Bestimmung der Teilchengröße von aminfunktionellen Oligoharnstoffen in der Nanodispersion mittels Zetasizer (Malvern) ergab das Maximum der Verteilungskurve bei 25 nm und die Verteilung von 14 bis 35nm. Die Teilchengrößenverteilungskurve ist in Fig. 2 dargestellt.

Die Nanodispersion wies eine Hydroxylzahl von 445 mg KOH/g, eine Aminzahl von 115 mg KOH/g und eine Viskosität (Rotation) von 1090 mPas (25°C) auf.

### Beispiel 8

Synthese einer Nanodispersion, die aminofunktionelle und biofunktionelle Mono-und Oligoharnstoffe aus 4,4'-MDI und N-Aminoethylpiperazin (NAEP) und Diethyltriamin (DETA) enthält, in einem Edelstahlreaktor (Zweistufenverfahren). Synthese des Vorprodukts (1. Stufe): In einen 10 l doppelwandigen Edelstahlreaktor mit Rührer, Beheizung mit Thermalöl, Dosierungen für Flüssigkeiten und Feststoffe, Stickstoffeinleitung und Wärmetauscher wurden 2,5 kg DMD gegeben, auf 45°C erwärmt und aufgeschmolzen. Zu dem flüssigen Isocyanat wurden langsam 5,5 kg Lupranol® 1100unter Rühren zugegeben, so dass die Temperatur 55°C nicht überstieg. Das Gemisch wurde anschließend bei 46°C noch ca. 1 h gerührt. Es entstand ein homogenes, zähfließendes, gelbliches Produkt mit dem Isocyanatgehalt 5,32 %.

Umsetzung des Vorproduktes (2.Stufe): In einen 20 l Edelstahlreaktor mit Rührer, Beheizung mit Thermalöl, Dosierungen für Flüssigkeiten und Feststoffe, Stickstoffeinleitung und Wärmetauscher wurden 4,14 kg Dipropylenglykol, 0,37 kg Diethylentriamin und 0,67 kg N-Aminoethylpiperazin (NAEP) eingewogen und dieses Gemisch unter Rühren auf 160°C erwärmt. Innerhalb von 40 Minuten wurden 4,82 kg des oben hergestellten Vorprodukts zugegeben. Nach beendeter Zugabe wurde das Gemisch bei 180°C weitere 45 min gerührt. Das Reaktionsgemisch wurde mittels eines Bodenventils abgelassen. Es war homogen, leicht fließend, braun-orange und klar. Die Nanodispersion enthielt ca. 24% Oligoharnstoffe mit Aminendgruppen. Die Bestimmung der Teilchengröße von aminfunktionellen Oligoharnstoffen in der Nanodispersion mittels Zetasizer (Malvern) ergab das Maximum der Teilchengrößenverteilungskurve bei 5,2nm und eine Verteilung von 3 bis 8 nm. Die Nanodispersion wies eine Hydroxylzahl von 434 mg KOH/g, eine Aminzahl von 102 mg KOH/g und eine Viskosität (Rotation) von 1860 mPas (25°C) auf. Die Nanodispersion besitzt biozide Wirkung gegen Algen, Daphnien und Bakterien. Sie kann zur Herstellung von Beschichtungen mit biozider Wirkung eingesetzt werden.

### Beispiel 9

Einstufenverfahren: In einen 20 l doppelwandigen Edelstahlreaktor mit Rührer, Beheizung mit Thermalöl, Dosierungen für Flüssigkeiten und Feststoffe, Stickstoffeinleitung und Wärmetauscher wurden 1.5 kg DMD gegeben,
auf 45°C erwärmt und aufgeschmolzen. Zu dem flüssigen Isocyanat wurde ein Gemisch aus 3,44 kg Lupranol® 1100, 4,3kg DPG und 0,7kg DETA unter Rühren zugegeben. Gleichzeitig wurde das Gemisch unter Rühren auf 180°C erwärmt und bei dieser Temperatur 30 Minuten gerührt. Das Reaktionsgemisch wurde mittels eines Bodenventils abgelassen. Es war homogen, leicht fließend, gelblich und optisch klar. Die Nanodispersion enthielt ca. 22,6 % Oligoharnstoffe mit Aminendgruppen. Die Bestimmung der Teilchengröße von aminfunktionellen Oligoharnstoffen in der Nanodispersion mittels Nanophox® (Sympatec GmbH, PCCS) und Zetasizer S (Malvern) ergab das Maximum der Teilchengrößenverteilungskurve bei 12 nm und die Verteilung von 8 bis 15 nm. Die Nanodispersion wies eine Hydroxylzahl von 457 mg KOH/g, eine Aminzahl von 78 mg KOH/g und eine Viskosität (Rotation) von 851 mPas (25°C) auf.

Die Nanodispersion kann zur Herstellung von Beschichtungen eingesetzt werden.

### Beispiel 10

Synthese des Vorprodukts (1. Stufe): In einen 10 l doppelwandigen Edelstahlreaktor mit Rührer, Beheizung mit Thermalöl, Dosierungen für Flüssigkeiten und Feststoffe, Stickstoffeinleitung und Wärmetauscher wurden 2,5 kg DMD gegeben, auf 45°C erwärmt und aufgeschmolzen. Zu dem flüssigen Isocyanat wurden langsam 5,5 kg Lupranol® 1100 unter Rühren zugegeben, so dass die Temperatur 55°C nicht überstieg. Das Gemisch wurde anschließend bei 46°C noch ca. 1 h gerührt. Es entstand ein homogenes, zähfließendes, gelbliches Produkt mit dem Isocyanatgehalt 5,32 %.

Umsetzung des Vorproduktes (2.Stufe): In einen 20 l Edelstahlreaktor mit Rührer, Beheizung mit Thermalöl, Dosierungen für Flüssigkeiten und Feststoffe, Stickstoffeinleitung und Wärmetauscher wurden 3,95 kg DPG, 0,83 kg DETA und 0,64 kg TCD eingewogen und dieses Gemisch unter Rühren auf 160°C erwärmt. Innerhalb von 20 Minuten wurden 4,58 kg des oben hergestellten Vorproduktes zugegeben. Nach beendeter Zugabe wurde das Gemisch bei 180°C weitere 30 min gerührt. Das Reaktionsgemisch wurde mittels eines Bodenventils abgelassen. Es war homogen, leicht dünnflüssig, gelb und klar. Die Nanodispersion enthielt ca. 30 Gew.% Oligoharnstoffen mit Aminendgruppen. Die Bestimmung der Teilchengröße von aminfunktionellen Oligoharnstoffen in der Nanodispersion mittels Zetasizer (Malvern) ergab das Maximum der Teilchengrößenverteilungskurve bei 4,7 nm und eine Verteilung von 3 bis 8 nm. Die Nanodispersion wies eine Hydroxylzahl von 496 mg KOH/g, eine Aminzahl von 143 mg KOH/g und eine Viskosität (Rotation) von 707 mPas (25°C) auf.

### Beispiel 11

In einen 250ml Sulfierkolben (Einschrittverfahren. Intensivkühler, Stickstoffzuführung und Temperatursensor, Magnetrührer) wurden 15,6 g DMD unter Stickstoffbegasung aufgeschmolzen (47°C) und danach ein Gemisch aus 4 g DETA, 6 g NAEP, 34,4 g Lupranol 1100 und 40 g DPG zugesetzt und unter Temperaturerhöhung auf 180°C gerührt. Das Gemisch verfestigte sich zunächst und ergab nach 30 Min Reaktionszeit ein optisch klares Produkt mit einem Oligoharnstoffgehalt von ca. 23 Gew.%, einer OHZ von 437, einer Aminzahl von 86 und einer Viskosität (Rotation) von 1850 mPas (25°C). Die Nanodispersion kann zur Herstellung von Beschichtungen eingesetzt werden.

### Beispiel 12

Synthese einer Nanodispersion, die aminofunktionelle Mono- und Oligoharnstoffe enthält, aus Vorastar HB 6549, DETA im Sulfierkolben (Einschrittverfahren).

In einen 250ml Sulfierkolben (Intensivkühler, Stickstoffzuführung und Temperatursensor, Magnetrührer) wurden 41,1 g DETA unter Rühren auf 160°C erhitzt und unter Rühren 58,9 g Vorastar HB 6549 zugesetzt. Nach 60 Min Reaktionszeit bei 180°C entstand ein gelbes, homogenes, leicht trübes Produkt, das ca. 50% Oligoharnstoffe enthält, mit einer Viskosität von 7420 mPas (25°C), einer OHZ von 548 und einer Aminzahl von 428. Die Nanodispersion kann zur Herstellung von Beschichtungen eingesetzt werden.

### Beispiel 13

In einen 250 ml Sulfierkolben (Zweischritttverfahren, Intensivkühler, Stickstoffzuführung und Temperatursensor, Magnetrührer) wurden 50 g DPG und 10 g DETA auf 180°C unter Rühren erhitzt. Zu dem Gemisch wurden 10 g eines Polymers aus trimerisiertem HDI und einer tetrafunktionellen SH-funktionellen Verbindung (Pentaerythritol-tetra(3-mercaptopropionat), THIOCURE® PETMP, BRUNO BOCK Chemische Fabrik GmbH & Co. KG) gegeben. Das Gemisch wurde auf 210°C erhitzt und 1 Stunde gerührt. Nach 60 Minuten Reaktionszeit entstand ein braunes, optisch klares, homogenes Produkt mit einem theoretischen Oligoharnstoffgehalt von 12% und einer Viskosität von 864 mPas. Die Nanodispersion kann zur Herstellung von Beschichtungen eingesetzt werden.

### Beispiel 14

In einen 250 ml Sulfierkolben (Zweischrittverfahren, Intensivkühler, Stickstoffzuführung und Temperatursensor, Magnetrührer) wurden 20 g DETA auf 180°C unter Rühren erhitzt. Zum DETA wurden 15,27 g eines Polymers aus trimerisiertem HDI (Desomdur N 3900) und einer tetrafunktionellen SH-funktionellen Verbindung (Pentaerythritol-tetra(3-mercaptopropionat), THIOCURE® PETMP, BRUNO BOCK Chemische Fabrik GmbH & Co. KG) gegeben. Das Gemisch wurde auf 205°C erhitzt und 1,5 Stunden gerührt. Nach der Reaktionszeit entstand ein braunes, optisch klares, homogenes Produkt mit einem theoretischen Oligoharnstoff-Gehalt von 36,8 Gew.% und einer Viskosität von 1500 mPas. Die Nanodispersion kann zur Herstellung von Beschichtungen eingesetzt werden.

### Beispiel 15

In einen 250ml Sulfierkolben (Zweischritttverfahren, Intensivkühler, Stickstoffzuführung und Temperatursensor, Magnetrührer) wurden 50 g DPG und 10 g Adipinsäure auf 180°C unter Rühren erhitzt. Zu dem Gemisch wurden 10 g eines Polymers aus trimerisiertem HDI und einer tetrafunktionellen SH-funktionellen Verbindung gegeben. Das Gemisch wurde auf 210°C erhitzt und 2 Stunden gerührt. Nach der Reaktionszeit entstand ein gelbes, optisch klares, homogenes Produkt mit einer Viskosität von 820 mPas. Die Nanodispersion kann zur Herstellung von Beschichtungen eingesetzt werden.

### Beispiel 16 (zweiphasig)

Synthese des Prepolymeren: In einem 10 l Edelstahlreaktor mit Rührer, Beheizung mit Thermalöl, Dosierungen für Flüssigkeiten und Feststoffe, Stickstoffeinleitung und Wärmetauscher wurden 2,5 kg DMD auf 45°C erwärmt und dabei aufgeschmolzen und dieses langsam unter Rühren mit 10 kg Lupranol® 1000 vermischt. Das Gemisch wurde anschließend bei 46°C ca. 1 h gerührt. Es entstand ein homogenes, zähfließendes, gelbliches, leicht trübes Prepolymer mit einem Isocyanatgehalt von 4,13 %.

Umsetzung des Prepolymeren: In einen 12,5 l Edelstahlreaktor mit Rührer, Beheizung mit Thermalöl, Dosierungen für Flüssigkeiten und Feststoffe, Stickstoffeinleitung und Wärmetauscher wurden 3,2 kg DPG, 0,8 kg DETA und 0,5 kg DBA eingewogen und dieses Gemisch unter Rühren auf 160°C erwärmt. Innerhalb von 60 Minuten wurden 5,5 kg des oben hergestellten Prepolymeren zugegeben.

Nach beendeter Zugabe wurde das Gemisch bei 180°C weitere 30 min gerührt. Das Reaktionsgemisch wurde mittels eines Bodenventils abgelassen. Es ist nach 24 Stunden Standzeit bei Raumtemperatur zweiphasig. Die Dispersion (klare untere Phase) war homogen, fließend und bei 65°C klar. Die Dispersion enthielt 20 Gew.% aminfunktionelle Harnstoffpartikel. Die Bestimmung der Teilchengröße der aminfunktionellen Oligoharnstoffe im reaktiven Sol mittels Nanophox® (Sympatec GmbH, PCCS) ergab das Maximum der Verteilungskurve bei 11 nm und die Verteilung von 9 bis 15 nm. Die Nanodispersion wies eine Hydroxylzahl von 653 mg KOH/g, eine Aminzahl von 198 mg KOH/g und eine Viskosität (Rotation) von 390 mPas (25°C) auf

## Patentansprüche

1. Verfahren zur Herstellung von oligomeren Harnstoff-Verbindungen durch Umsetzung von Ausgangsverbindungen mit jeweils zumindest zwei reaktiven Gruppen, ausgewählt aus der Hydroxy- und/oder Thiol- Gruppe, mit Di- oder Polyisocyanten
bei einer ersten Reaktionstemperatur, um Polyurethan- und/oder Polythiourethan-Verbindungen aufzubauen (Kettenaufbau),
und nachfolgende Depolymerisation der entstandenen Polyurethan- und/oder Polythiourethan-Verbindungen (Kettenabbau) in Anwesenheit eines primären
oder sekundären Di- oder Polyamins, aufweisend in der Summe pro Molekül zumindest zwei primäre, zumindest zwei sekundäre oder zumindest eine primäre und zumindest eine sekundäre Amin-Gruppe, bei einer zweiten Reaktionstemperatur, wobei die zweite Reaktionstemperatur, bezogen auf das Maximum der jeweiligen Reaktionstemperatur, um mindestens 40°C höher ist, als die erste Reaktionstemperatur, um Zusammensetzungen zu erhalten, aufweisend oligomere Harnstoff-Verbindungen und die Ausgangsverbindungen mit zumindest zwei reaktiven Gruppen, ausgewählt aus der Hydroxy- und/oder Thiol-Gruppe, und wobei
a) die Polyurethan- und/oder Polythiourethan-Verbindungen unter den Bedingungen der Umsetzung stets als Flüssigkeit, in einer Flüssigkeit dispergiert oder in gelöster Form vorliegen, oder
b) bei der Umsetzung Kettenaufbau und Depolymerisation in einem einzigen Reaktor durchgeführt werden oder
c) a) und b) gleichzeitig vorliegen.

2. Verfahren nach Anspruch 1, wobei Zusammensetzungen erhalten werden aufweisend oligomere Harnstoff-Verbindungen mit einem Oligomersationsgrad von 2 bis 16 und unabhängig hiervon, bezogen auf die Oligoharnstoff-Moleküle der oligomeren Harnstoff-Verbindungen und ausgenommen die Monomere, mehr als 50 %, insbesondere mehr als 80 %, aller Oligoharnstoff-Moleküle Oligomerisationsgrade von 2 bis 16, insbesondere 2 bis 8 aufweisen.

3. Verfahren nach Anspruch 1 oder 2, wobei die Herstellung der Polyurethan-und/oder Polythiourethan-Verbindungen in Abwesenheit von Wasser, Polyurethankatalysatoren, Farbstoffen, Stabilisatoren und/oder Treibmitteln, insbesondere in Abwesenheit von Polyurethankatalysatoren, Farbstoffen, Stabilisatoren und/oder Treibmitteln, erfolgt.

4. Verfahren nach zumindest einem der vorhergehenden Ansprüche, wobei ein ein Dispergiermittel zumindest während der Depolymerisation zugesetzt wird.

5. Verfahren nach Anspruch 4, wobei das Dispergiermittel ein Di- oder Polyol mit 2 bis 40 Kohlenstoffatomen ist.

6. Verfahren nach zumindest einem der vorhergehenden Ansprüche, wobei die Umsetzung zum Kettenaufbau bei 20 bis 120°C (erste Reaktionstemperatur) und die Depolymerisation bei größer 120, vorzugsweise größer 150°C bis 250°C (zweite Reaktionstemperatur) durchgeführt wird und ggf. unabhängig hiervon vorzugsweise die zweite Reaktionstemperatur um mindestens 70°C, insbesondere mindestens 100°C höher ist, als die erste Reaktionstemperatur.

7. Verfahren nach zumindest einem der vorhergehenden Ansprüche, wobei die oligomeren Harnstoff-Verbindungen mit einer oder mehreren reaktiven Gruppen von Fungizid-, Biozid- oder Herbizid-Verbindungen umgesetzt werden und ggf. unabhängig hiervon vorzugsweise die primären und/oder sekundären Di- oder Polyamine zumindest teilweise Fungizid-, Biozid- oder Herbizid-Verbindungen sind.

8. Verfahren nach zumindest einem der vorhergehenden Ansprüche, wobei die Oligoharnstoff-Moleküle der oligomeren Harnstoff-Verbindungen zumindest teilweise in Teilchenform und dispergiert vorliegen.

9. Verfahren nach zumindest einem der vorhergehenden Ansprüche, wobei die primären oder sekundären Di- oder Polyamine und ggf. zusätzlich Mono-Amine, Wasser, Harnstoff und/oder Ammoniak nach Bildung der Polyurethan- und/oder Polythiourethan-Verbindungen und vor oder während der Temperaturerhöhung auf die zweite Reaktionstemperatur zugegeben werden, vorzugsweise vor der Temperaturerhöhung auf die zweite Reaktionstemperatur.

10. Oligoharnstoff-Dispersionen, enthaltend ein Dispergiermittel und oligomere Harnstoff-Verbindungen mit 2 bis 16, vorzugsweise 2 bis 8 Monomereinheiten, insbesondere im Mittel (ausgenommen Monomere) mit 2 bis 16, vorzugsweise im Mittel 2 bis 8, Monomereinheiten,
wobei insbesondere mehr als 50 %, insbesondere mehr als 80 %, aller Oligoharnstoff-Moleküle der oligomere Harnstoff-Verbindungen (ausgenommen die Monomere) Oligomerisationsgrade von 2 bis 16, insbesondere 2 bis 8 aufweisen, wobei die Oligoharnstoff-Moleküle in dem Dispergiermitttel Teilchengrößen von kleiner 40 nm für 90% aller Teilchen aufweisen.

11. Oligoharnstoff-Dispersionen nach Anspruch 10 enthaltend ein Dispergiermittel und Oligoharnstoff-Moleküle der oligomeren Harnstoff-Verbindungen (ausgenommen Monomere) mit Teilchengrößen von im Mittel 4 bis 40 nm, vorzugsweise 8 bis 20 nm.

12. Oligoharnstoff-Dispersionen nach Anspruch 10 oder 11, wobei die oligomeren Harnstoff-Verbindungen Endgruppen aufweisen ausgewählt aus mindestens einer freien Amino- und/oder Hydroxylgruppe, Carboxyl-, oder SH-Gruppe als Endgruppe.

13. Oligoharnstoff-Dispersionen nach Anspruch 10 oder 11, wobei das Dispergiermittel pro Molekül zwei freie / funktionelle Gruppen aufweist, ausgewählt aus der Gruppe -OH, -NH₂ und/oder =NH, vorzugsweise ganz oder teilweise -OH.

14. Oligoharnstoff-Dispersionen nach zumindest einem der Ansprüche 10 bis 13, wobei die Zusammensetzung unabhängig voneinander enthält:
(a) 1 bis 90 Gew.-% oder 5 bis 50 Gew.-% Mono- und/oder Oligoharnstoff-Moleküle, und
99 bis 10 Gew.-%, oder 95 bis 50 Gew.-%, Dispergiermittel; und / oder
(b) größer 20 Gew.%, vorzugsweise größer 30 Gew.%, Oligoharnstoff-Teilchen.

15. Oligoharnstoff-Dispersionen nach zumindest einem der Ansprüche 10 bis 14, wobei die Oligoharnstoff-Dispersion nach einem der Verfahren nach Anspruch 1 bis 9 erhältlich ist.

16. Verwendung der oligomeren Harnstoff-Verbindungen der Oligoharnstoff-Dispersionen nach zumindest einem der Ansprüche 10 bis 15 als Bestandteil von Fungiziden, Bioziden und/oder Herbiziden.

## Claims

1. A method for producing oligomeric urea compounds by reacting starting compounds with at least two reactive groups each, selected from hydroxy and/or thiol groups, with di- or polyisocyanates
at a first reaction temperature to produce polyurethane and/or polythiourethane compounds (chain elongation),
and subsequent depolymerization of the resulting polyurethane and/or polythiourethane compounds (chain degradation) in the presence of a primary or secondary di- or polyamine, containing in total per molecule at least two primary, at least two secondary or at least one primary and at least one secondary amine group, at a second reaction temperature,
wherein the second reaction temperature, in respect of the maximum of each of the reaction temperatures, is at least 40°C higher than the first reaction temperature, in order to obtain compositions having oligomeric urea compounds and the starting compounds with at least two reactive groups selected from the hydroxy and/or thiol group, and wherein
a) the polyurethane and/or polythiourethane compounds under the reaction conditions are always present as liquids, dispersed in a liquid or in dissolved form, or
b) in the reaction, chain elongation and depolymerization are carried out in a single reactor, or
c) a) and b) apply simultaneously.

2. The method according to claim 1, wherein compositions are obtained having oligomeric urea compounds with a degree of oligomerization of 2 to 16 and independently thereof, based on the oligourea molecules of the oligomeric urea compounds, and with the exception of the monomers, more than 50 %, especially more than 80 %, of all oligourea molecules have degrees of oligomerization of 2 to 16, especially 2 to 8.

3. The method according to claim 1 or 2, wherein the production of the polyurethane and/or polythiourethane compounds takes place in the absence of water, polyurethane catalysts, dyes, stabilizers and/or blowing agents, especially in the absence of polyurethane catalysts, dyes, stabilizers and/or blowing agents.

4. The method according to any one or more of the preceding claims, wherein a dispersant is added at least during depolymerization.

5. The method according to claim 4, wherein the dispersant is a diol or polyol with 2 to 40 carbon atoms.

6. The method according to any one or more of the preceding claims, wherein the reaction for chain elongation is performed at 20 to 120°C (first reaction temperature) and the depolymerization at greater than 120, advantageously greater than 150°C to 250°C (second reaction temperature), and optionally independently thereof, the second reaction temperature is advantageously at least 70°C, especially at least 100°C higher than the first reaction temperature.

7. The method according to any one or more of the preceding claims, wherein the oligomeric urea compounds are reacted with one or more reactive groups of fungicidal, biocidal or herbicidal compounds, and optionally independent thereof advantageously the primary and/or secondary diamines or polyamines are at least in parts fungicidal, biocidal or herbicidal compounds.

8. The method according to any one or more of the preceding claims, wherein the oligourea molecules of the oligomeric urea compounds are present at least partially in particulate form and dispersed.

9. The method according to any one or more of the preceding claims, wherein the primary or secondary diamines or polyamines and optionally further monoamines, water, urea and/or ammonia, are added after formation of the polyurethane and/or polythiourethane compounds and before or during the temperature increase to the second reaction temperature, advantageously before temperature increase to the second reaction temperature.

10. Oligourea dispersions containing a dispersant and oligomeric urea compounds with 2 to 16, advantageously 2 to 8 monomer units, especially on average (excluding monomers) with 2 to 16, advantageously on average 2 to 8, monomer units,
wherein especially more than 50 %, especially more than 80 %, of all oligourea molecules of the oligomeric urea compounds (with the exception of the monomers) have degrees of oligomerization of 2 to 16, especially 2 to 8, wherein the oligourea molecules in the dispersants have particle sizes of less than 40 nm for 90% of all particles.

11. Oligourea dispersions in accordance with claim 10, containing a dispersant and oligourea molecules of the oligomeric urea compounds (with the exception of monomers) with average particle sizes of 4 to 40 nm, advantageously 8 to 20 nm.

12. Oligourea dispersions in accordance with claim 10 or 11, wherein the oligomeric urea compounds have end groups selected from at least one free amino and/or hydroxyl group, carboxyl, or SH group as the end group.

13. Oligourea dispersions in accordance with claim 10 or 11, wherein the dispersant per molecule has at least two free/ functional groups selected from the groups -OH, -NH₂ and/or =NH, advantageously with all or at least some of them -OH groups.

14. Oligourea dispersions according to any one or more of claims 10 to 13, wherein the composition contains, independently of one another:
(a) 1 to 90 % by weight or 5 to 50 % by weight mono- and/or oligourea molecules, and
99 to 10 % by weight, or 95 to 50 % by weight, dispersant; and / or
(b) more than 20 % by weight, advantageously more than 30 % by weight, oligourea particles.

15. Oligourea dispersions according to any one or more of claims 10 to 14, wherein the oligourea dispersion can be obtained by a method according to any one of claims 1 to 9.

16. Use of the oligomeric urea compounds of the oligourea dispersions according to at least one of claims 10 to 15 as a component of fungicides, biocides and/or herbicides.

## Revendications

1. Procédé de fabrication de composés d'urée oligomères par réaction de composés de départ chacun avec au moins deux groupes réactifs, choisis parmi le groupe hydroxy et/ou thiol, avec des di- ou polyisocyanates
à une première température de réaction, de façon à construire des composés polyuréthanes et/ou polythiouréthanes (construction de chaîne),
et dépolymérisation subséquente des composés polyuréthanes et/ou polythiouréthanes résultants (dégradation de chaîne) en présence d'une di- ou polyamine primaire ou secondaire, présentant dans la somme par molécule au moins deux groupes amine primaire, au moins deux groupes amine secondaire ou au moins un groupe amine primaire et au moins un groupe amine secondaire, à une seconde température de réaction, la seconde température de réaction, par rapport au maximum de la température de réaction respective, étant supérieure d'au moins 40°C à la première température de réaction, de façon à obtenir des compositions présentant des composés d'urée oligomères et les composés de départ avec au moins deux groupes réactifs, choisis parmi le groupe hydroxy et/ou thiol, et où
a) les composés polyuréthaned et/ou polythiouréthaned dans les conditions de la réaction sont constamment présents sous la forme d'un liquide, dispersés dans un liquide ou dans une forme dissoute, ou
b) lors de la réaction la construction de chaîne et la dépolymérisation sont effectuées dans un seul réacteur, ou
c) a) et b) ont lieu simultanément.

2. Procédé selon la revendication 1, dans lequel des compositions sont obtenues présentant des composés d'urée oligomères avec un degré d'oligomérisation de 2 à 16 et indépendamment de ceci, par rapport aux molécules d'oligourée des composés d'urée oligomères et à l'exclusion des monomères, plus de 50 %, en particulier plus de 80 %, de toutes les molécules d'oligourée présentent des degrés d'oligomérisation de 2 à 16, en particulier de 2 à 8.

3. Procédé selon l'une des revendications 1 ou 2, dans lequel la fabrication des composés polyuréthanes et/ou polythiouréthanes a lieu en l'absence d'eau, de catalyseurs de polyuréthane, de colorants, de stabilisants et/ou d'agents gonflants, en particulier en l'absence de catalyseurs de polyuréthane, de colorants, de stabilisants et/ou d'agents gonflants.

4. Procédé selon au moins l'une des revendications précédentes, dans lequel un dispersant est ajouté au moins pendant la dépolymérisation.

5. Procédé selon la revendication 4, dans lequel le dispersant est un di- ou polyol ayant 2 à 40 atomes de carbone.

6. Procédé selon au moins l'une des revendications précédentes, dans lequel la réaction de construction de chaîne est effectuée à 20 à 120°C (première température de réaction) et la dépolymérisation est effectuée à plus de 120, de préférence à plus 150°C à 250°C (seconde température de réaction) et le cas échéant indépendamment de ceci de préférence la seconde température de réaction est supérieure d'au moins 70°C, en particulier supérieure d'au moins 100°C à la première température de réaction.

7. Procédé selon au moins l'une des revendications précédentes, dans lequel les composés d'urée oligomères sont mis à réagir avec un ou plusieurs groupes réactifs de composés fongicides, biocides ou herbicides et le cas échéant indépendamment de ceci de préférence les di-ou polyamines primaires et/ou secondaires sont au moins partiellement des composés fongicides, biocides ou herbicides.

8. Procédé selon au moins l'une des revendications précédentes, dans lequel les molécules d'oligourée des composés d'urée oligomères se présentent au moins partiellement sous forme de particules et dispersées.

9. Procédé selon au moins l'une des revendications précédentes, dans lequel les di- ou polyamines primaires ou secondaires et le cas échéant de plus des mono-amines, de l'eau, de l'urée et/ou de l'ammoniac sont ajoutés après formation des composés polyuréthanes et/ou polythiouréthanes et avant ou pendant l'élévation de température à la seconde température de réaction, de préférence avant l'élévation de température à la seconde température de réaction.

10. Dispersion d'oligourée, contenant un dispersant et des composés d'urée oligomères ayant 2 à 16, de préférence 2 à 8 unités monomères, en particulier en moyenne (à l'exception des monomères), ayant 2 à 16, de préférence en moyenne 2 à 8, unités monomères,
où en particulier plus de 50 %, en particulier plus de 80 %, de toutes les molécules oligourée des composés d'urée oligomères (à l'exception des monomères) présentent des degrés d'oligomérisation de 2 à 16, en particulier de 2 à 8, les molécules d'oligourée dans le dispersant présentant des grosseurs de particule de moins de 40 nm pour 90 % de toutes les particules.

11. Dispersions d'oligourée selon la revendication 10, contenant un dispersant et des molécules d'oligourée des composés d'urée oligomères (à l'exclusion des monomères) ayant des grosseurs de particule d'en moyenne 4 à 40 nm, de préférence 8 à 20 nm.

12. Dispersion d'oligourée selon l'une des revendications 10 ou 11, dans laquelle les composés d'urée oligomères présentent des groupes terminaux choisis parmi au moins un groupe amino et/ou hydroxyle libre, un groupe carboxyle ou SH comme groupe terminal.

13. Dispersions d'oligourée selon l'une des revendications 10 ou 11, dans lesquelles le dispersant présente par molécule deux groupes libres/fonctionnels, choisis dans le groupe -OH, -NH₂ et/ou =NH, de préférence totalement ou partiellement -OH.

14. Dispersions d'oligourée selon au moins l'une des revendications 10 à 13, dans lesquelles la composition contient indépendamment l'un de l'autre :
(a) 1 à 90 % en poids ou 5 à 50 % en poids de molécules de mono- et/ou oligourée ; et
99 à 10 % en poids, ou 95 à 50 % en poids, de dispersant ; et/ou
(b) plus de 20 % en poids, de préférence plus de 30 % en poids de particules d'oligourée.

15. Dispersions d'oligourée selon au moins l'une des revendications 10 à 14, dans lesquelles la dispersion d'oligourée est susceptible d'être obtenue par l'un des procédés selon l'une des revendications 1 à 9.

16. Utilisation des composés d'urée oligomères des dispersions d'oligourée selon au moins l'une des revendications 10 à 15 comme constituant de fongicides, biocides et/ou herbicides.
